# EUROPEAN PATENT APPLICATION

(11) **EP 4 586 265 A1**
(43) Date of publication of application: **16.07.2025**
(21) Application number: 23877696.7
(22) Date of filing: 12.10.2023
(51) Int. Cl.: G16H 30/00, G16H 20/00, G16H 50/70, G06V 10/82, G06V 10/25, G06V 10/46, G06V 10/50

(54) **METHOD, PROGRAM, AND APPARATUS FOR SEARCHING FOR BLOOD COLLECTION CANDIDATE**

(30) Priority: 14.10.2022 KR 20220132184
(71) Applicant: Airs Medical Inc., Seoul 08788 (KR)
(72) Inventor: KIM, Youngsik, Seoul 06571 (KR)
(74) Representative: Isarpatent
(86) International application number: PCT/KR2023/015694
(87) International publication number: WO 2024/080773

(57) **Abstract**

There are disclosed a method, program, and apparatus for searching for a blood collection candidate according to one embodiment of the present disclosure. The method may include: detecting a blood vessel present in a medical image by using a pre-trained deep learning model; and searching for bounding boxes, including at least a part of the detected blood vessel, based on the workspace of a medical robot, and determining a blood collection candidate.

## Description

### Technical Field

The present disclosure relates to medical image processing technology, and more particularly, to a method and apparatus that search for the target location of a blood vessel present in a medical image.

### Background Art

Blood collection refers to an invasive medical procedure that is performed on a blood vessel for the purpose of diagnosing a disease, performing a blood transfusion, or the like. Blood collection is generally performed by identifying a vein close to the surface of the skin of a subject with the naked eye and inserting a needle into a blood vessel with the needle targeted at the vein. In other words, blood collection is generally performed manually and individually by a person. Accordingly, blood collection has a problem in that the blood collection time and success rate can vary considerably depending on the skill of the person who performs blood collection. Furthermore, blood collection has a problem in that there is a possibility that infection can occur through human contact during an invasive process for blood collection.

In order to overcome the above problems of blood collection, apparatuses for automating a blood collection process have been developed recently. Blood collection apparatuses aim to perform the procedures, required to be performed for blood collection and ranging from the preparation for blood collection through the discovery of a blood vessel to the insertion of a needle, stably through apparatuses rather than people. In order to achieve this aim, it is most important to specify the location of a blood vessel that is the target of blood collection.

Meanwhile, medical images are data that allow users to understand the physical states of various organs in the human body for the diagnosis and treatment of diseases. As medical imaging apparatuses continue to be developed, medical images are diversifying into X-ray images, computed tomography (CT) images, positron emission tomography (PET) images, magnetic resonance imaging (MRI) images, etc. With the development of image analysis technology and artificial intelligence technology, medical images are being utilized in various manners as analysis data for assistance with medical techniques. Typically, medical images are actively utilized in the field of detection in which objects such as blood vessels captured in images are searched for such that medical techniques can be performed effectively.

### Disclosure

### Technical Problem

The present disclosure has been conceived in response to the above-described background art, and is intended to provide a method and apparatus that search for the location of a blood vessel suitable for blood collection by analyzing a medical image based on deep learning and recommend the results of the searching.

However, the objects to be achieved in the present disclosure are not limited to the object mentioned above, and other objects not mentioned may be clearly understood based on the following description.

### Technical Solution

According to one embodiment of the present disclosure for achieving the above-described object, there is disclosed a method for searching for a blood collection candidate that is performed by a computing device. The method may include: detecting a blood vessel present in a medical image by using a pre-trained deep learning model; and searching for bounding boxes, including at least a part of the detected blood vessel, based on the workspace of a medical robot, and determining a blood collection candidate.

Alternatively, detecting the blood vessel present in the medical image by using the pre-trained deep learning model may include: generating a first binary corresponding to a blood vessel present in the medical image by using a first sub-model included in the deep learning model; and generating a second binary corresponding to a blood vessel present in the medical image by using a second sub-model included in the deep learning model. In this case, the second sub-model may be pre-trained to detect a superficial vein by binarizing a medical image.

Alternatively, detecting the blood vessel present in the medical image by using the pre-trained deep learning model may further include: applying a weight to the second binary; and generating a third binary to be used for determining the blood collection candidate by combining the second binary to which the weight is applied and the first binary.

Alternatively, searching for the bounding boxes, including at least a part of the detected blood vessel, based on the workspace of the medical robot, and determining the blood collection candidate may include: searching for bounding boxes present inside the detected blood vessel and equal to or larger than a predetermined size within an operation limit angle of the medical robot; and determining the bounding box equal to or larger than the predetermined size to be a blood collection candidate.

Alternatively, searching for the bounding boxes including at least a part of the detected blood vessel and equal to or larger than the predetermined size within the operation limit angle of the medical robot may include: searching for bounding boxes while rotating the detected blood vessel within the operation limit angle of the medical robot; and selecting a bounding box equal to or larger than a predetermined size from among the bounding boxes found while rotating the detected blood vessel.

Alternatively, searching for the bounding boxes while rotating the detected blood vessel within the operation limit angle of the medical robot and selecting the bounding box equal to or larger than the predetermined size from among the bounding boxes found while rotating the detected blood vessel may be repeatedly performed until the number of selected bounding boxes becomes equal to or larger than a first reference value.

Alternatively, detecting the blood vessel present in the medical image by using the pre-trained deep learning model may include: generating a first binary corresponding to a blood vessel present in the medical image by using a first sub-model included in the deep learning model; and generating a third binary to be used for determining the blood collection candidate based on the first binary by using a second sub-model included in the deep learning model. In this case, the second sub-model may be pre-learned to detect a superficial vein based on a binarized medical image.

Alternatively, when it is predetermined that a largest bounding box is selected from among the bounding boxes found while rotating the detected blood vessel, searching for the bounding boxes while rotating the detected blood vessel within the operation limit angle of the medical robot and selecting the bounding box equal to or larger than the predetermined size from among the bounding boxes found while rotating the detected blood vessel may be repeatedly performed until a bounding box, in which the number of pixels is smaller than or equal to a second reference value, is selected.

Alternatively, the method may further include dividing the medical image into a plurality of tiles and performing histogram equalization for each of the tiles.

Alternatively, the method may further include adjusting the contrast of the medical image so that a blood vessel present in the medical image is emphasized.

According to one embodiment of the present disclosure for achieving the above-described object, there is disclosed a computer program stored in a computer-readable storage medium. The computer program performs operations for searching for a blood collection candidate when executed on one or more processors. In this case, the operations may include operations of: detecting a blood vessel present in a medical image by using a pre-trained deep learning model; and searching for bounding boxes, including at least a part of the detected blood vessel, based on the workspace of a medical robot, and determining a blood collection candidate.

According to one embodiment of the present disclosure for achieving the above-described object, there is disclosed a computing device for searching for a blood collection candidate. The computing device may include: a processor including at least one core; memory including program codes executable on the processor; and a network unit for acquiring a medical image. In this case, the processor may detect a blood vessel present in a medical image by using a pre-trained deep learning model, and may search for bounding boxes, including at least a part of the detected blood vessel, based on the workspace of a medical robot, and determine a blood collection candidate.

### Advantageous Effects

The present disclosure may provide the method and apparatus that search for the location of a blood vessel suitable for blood collection by analyzing a medical image based on deep learning and recommend the results of the searching.

### Description of Drawings

FIG. 1 is a block diagram of a computing device according to one embodiment of the present disclosure;
FIG. 2 is a block diagram showing a process of searching for a blood collection candidate according to one embodiment of the present disclosure;
FIGS. 3 and 4 are block diagrams showing a process of searching for a blood collection candidate according to an alternative embodiment of the present disclosure;
FIG. 5 shows an example of an image derived from a process of searching for a blood collection candidate according to one embodiment of the present disclosure;
FIG. 6 is a flowchart showing a method for searching for a blood collection candidate according to one embodiment of the present disclosure; and
FIG. 7 is a flowchart showing a process of searching for a blood collection candidate according to one embodiment of the present disclosure.

### Mode for Invention

Embodiments of the present disclosure will be described in detail below with reference to the accompanying drawings so that those having ordinary skill in the art of the present disclosure (hereinafter those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "acquiring" used herein may be understood to mean not only receiving data from an external device or a system over a wired/wireless communication network but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or a neural network set in which multiple neural networks are combined together.

The term "image" used herein may refer to multidimensional data including discrete image elements. In other words, "image" may be understood as a term referring to a digital representation of an object that can be seen by the human eye. For example, "image" may refer to multidimensional data including elements corresponding to pixels in a two-dimensional image. "Image" may refer to multidimensional data including elements corresponding to voxels in a three-dimensional image.

The foregoing descriptions of the terms are intended to help to understand the present disclosure. Accordingly, it should be noted that unless the above-described terms are explicitly described as limiting the content of the present disclosure, the terms in the content of the present disclosure are not used in the sense of limiting the technical spirit of the present disclosure.

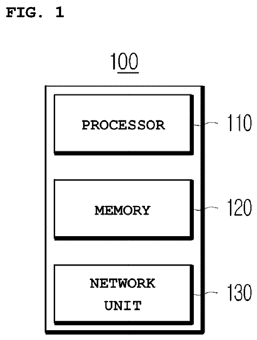

FIG. 1 is a block diagram of a computing device according to one embodiment of the present disclosure.

The computing device 100 according to one embodiment of the present disclosure may be a hardware device or a part of a hardware device that performs the comprehensive processing and computation of data, or may be a software-based computing environment connected over a communication network. For example, the computing device 100 may be a server that is a main agent for performing an intensive data processing function as a computing device and sharing resources, or may be a client that shares resources through interaction with a server. Furthermore, the computing device 100 may be a cloud system in which multiple servers and clients comprehensively process data while interacting with each other. Since the above description is only one example related to the type of computing device, the type of computing device may be configured in various manners within a range understandable to those skilled in the art based on the content of the present disclosure.

Referring to FIG. 1, the computing device 100 according to one embodiment of the present disclosure may include a processor 110, memory 120, and a network unit 130. However, FIG. 1 shows only an example, and accordingly, the computing device 100 may include other components for implementing a computing environment. Furthermore, only some of the components disclosed above may be included in the computing device 100.

The processor 110 according to one embodiment of the present disclosure may be understood as a constituent unit including hardware and/or software for performing computing operations. For example, the processor 110 may read a computer program and perform data processing for machine learning. The processor 110 may process operation processes such as the processing of input data for machine learning, the extraction of features for machine learning, and the computation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

The processor 110 may search for a blood vessel region suitable for blood collection based on a medical image by using a deep learning model. The processor 110 may detect a blood vessel present in a medical image by inputting the medical image to the deep learning model. For example, the processor 110 may detect a blood vessel in the forearm shown in an image by inputting the image, generated by an infrared camera, to the deep learning model. In this case, the deep learning model may include a neural network such as U-net that detects an object based on an image. Meanwhile, the functional expression 'the detection of the deep learning model' may be a concept that encompasses all tasks, such as detection, classification, and segmentation, that can be performed by a deep learning model in the computer vision field.

The processor 110 may determine a blood vessel region, suitable for blood collection from the blood vessels detected via the deep learning model, to be a blood collection candidate by considering the workspace of a medical robot. In the case where the movement for blood collection is limited due to the structure of the medical robot, when the blood collection candidate is determined without considering this limitation, a problem may occur in that blood collection cannot be performed via the medical robot even when the blood collection candidate determined by the processor 110 is suitable for actual blood collection. Accordingly, the processor 110 may determine the blood collection candidate by reflecting the operation limitation of the medical robot in the analysis of a medical image. For example, the processor 110 may search for bounding boxes, including at least a part of the blood vessel detected via the deep learning model in a medical image, by considering the operational limitation of the medical robot to the performance of blood collection. That is, the processor 110 may search for bounding boxes within the workspace where the medical robot can perform a blood collection operation by reflecting the workspace in the analysis of the medical image. In this case, the bounding box may be formed as one of polygons having various shapes such as an oval, a pentagon, a trapezoid, etc. as well as a box shape. The processor 110 may determine a blood collection candidate from the blood vessel detected via the deep learning model based on the bounding box found in the medical image.

Meanwhile, the processor 110 may perform preprocessing on the medical image to improve the learning and inference performance of the deep learning model. The processor 110 may remove a region unnecessary for the feature extraction of the deep learning model from the medical image or adjust the size of the medical image before inputting the medical image to the deep learning model. Furthermore, the processor 110 may adjust the pixel values of the medical image to facilitate visual distinction between a blood vessel and a surrounding region in the medical image. By performing the preprocessing as described above, the processor 110 may increase the efficiency and productivity of the deep learning model that detects a blood vessel in the medical image.

The memory 120 according to one embodiment of the present disclosure may be understood as a constituent unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize, and manage data required for the processor 110 to perform operations, combinations of data, and program codes executable by the processor 110. For example, the memory 120 may store medical images acquired via the network unit 130 to be described below. The memory 120 may store program codes intended to operate the deep learning model to be trained based on the medical images, program codes intended to operate the deep learning model to perform the feature interpretation (or the feature inference) of medical images, and various types of data generated as the program codes are executed.

The network unit 130 according to one embodiment of the present disclosure may be understood as a constituent unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, an ultra-wideband wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive data, required for the processor 110 to perform operations, through wired or wireless communication with any system, any client, or the like. Furthermore, the network unit 130 may transmit data, generated through the operations of the processor 110, through wired or wireless communication with any system, any client, or the like. For example, the network unit 130 may receive medical images through communication with a picture archiving and communication system, a cloud server for performing tasks such as the standardization of medical data, a medical robot, or the like. The network unit 130 may transmit various types of data, generated through the operations of the processor 110, through communication with the above-described system, server, or medical robot, or the like.

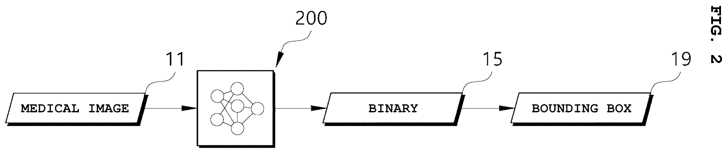

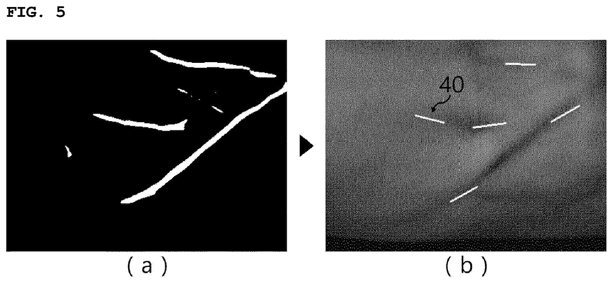

FIG. 2 is a block diagram showing a process of searching for a blood collection candidate according to one embodiment of the present disclosure. Furthermore, FIG. 5 shows an example of an image derived from a process of searching for a blood collection candidate according to one embodiment of the present disclosure.

The processor 110 of the computing device 100 according to one embodiment of the present disclosure may detect a blood vessel in a medical image 11 by inputting the medical image 11 to a deep learning model 200. The processor 110 may detect at least one blood vessel present in the medical image 11 by binarizing the medical image 11 using the deep learning model 200. The processor 110 may generate a binary 15 corresponding to at least one blood vessel present in the medical image 11 by inputting the medical image 11 to the deep learning model 200.

For example, when the computing device 100 acquires an infrared image of a blood vessel in the forearm as the medical image 11, the processor 110 may detect a blood vessel present in the infrared image by inputting the infrared image to the deep learning model 200 and binarizing the infrared image so that the blood vessel can be distinguished from the other regions in the infrared image. In other words, the deep learning model 200 may receive an infrared image and interpret its features, thereby generating the binary 15 corresponding to the blood vessel by classifying a blood vessel region present in the infrared image as 1 and the other regions excluding the blood vessel present in the infrared image as 0. In this case, the deep learning model 200 may be pre-trained to detect all blood vessel regions in training images by using labels that mark all blood vessel regions, present in infrared images, with ground truth (GT). The training images may be medical images including infrared images of blood vessels. More specifically, in the training process, the deep learning model 200 may receive a training image, may detect a blood vessel region in the image, and may perform binarization so that the detected region and the other regions can be distinguished from each other, thereby generating a binary corresponding to the blood vessel region. Furthermore, the deep learning model 200 may repeatedly perform a process of comparing the generated binary with labels and updating parameters based on the results of the comparison. Although the above-described example is a training process based on supervised learning, the deep learning model 200 may perform training through semi-supervised learning, unsupervised learning, self-supervised learning, or the like, other than supervised learning.

The processor 110 may extract a bounding box 19 that is present inside the blood vessel represented by the binary 15 within the operation limit angle of the medical robot and is equal to or larger than a predetermined size. More specifically, the processor 110 may rotate the blood vessel, represented by the binary 15, within the operation limit angle of the medical robot. The processor 110 may search for bounding boxes inside the blood vessel while rotating the blood vessel represented by the binary 15. The processor 110 may select the bounding box 19 equal to or larger than the predetermined size from among the bounding boxes found while rotating the blood vessel. For example, when the operation limit angle of the invasive part of the medical robot for blood collection ranges from -30 degrees to 30 degrees, the processor 110 may rotate the blood vessel, represented by the binary 15, within the range from -30 degrees to 30 degrees. Furthermore, the processor 110 may search for bounding boxes present in the binary 15 while rotating the binary 15 at all angles within the range from - 30 degrees to 30 degrees. The processor 110 may select the bounding box 19 equal to or larger than the predetermined size from among the found bounding boxes. In this case, the predetermined size may be a value that is set by the manufacturer or user of the medical robot to achieve the purpose of blood collection. That is, the size of the bounding box 19 selected for determining a blood collection candidate may be a value that is determined in advance by the manufacturer or user of the medical robot based on the clinical experience in which a thick and long blood vessel is suitable for blood collection. The size of the bounding box 19 selected for determining a blood collection candidate may be modified or changed by an external input that is applied through communication with the medical robot itself, a client terminal, or the like. Although the bounding box 19 for determining a blood collection candidate in an infrared image may be represented by a white box 40 as shown in FIG. 5(b), this is only an example. Accordingly, the shape and color of the bounding box 19 for determining a blood collection candidate may be configured in various manners in accordance with the preference of the manufacturer or user of the medical robot.

The processor 110 may determine the bounding box 19 equal to or larger than the predetermined size to be a blood collection candidate. Since the bounding box 19 equal to or larger than the predetermined size corresponds to a region determined to be a blood vessel suitable for blood collection based on the clinical experience of the manufacturer or user of the medical robot, the processor 110 may select the bounding box 19 equal to or larger than the predetermined size to be a blood collection candidate. The processor 110 may repeatedly perform an operation for selecting the bounding box 19 equal to or larger than the predetermined size until the number of bounding boxes 19 equal to or larger than the predetermined size becomes equal to or larger than a reference value. In this case, the reference value is a value pre-determined by the manufacturer or user of the medical robot, and may be modified or changed by an external input applied through communication with the medical robot itself, a client terminal, or the like. For example, the processor 110 may repeatedly perform the overall operation process of rotating the binary 15, searching for bounding boxes, and selecting a bounding box equal to or larger than the predetermined size within the operation limit angle of the medical robot until the number of bounding boxes 19 equal to or larger than the predetermined size becomes five or more.

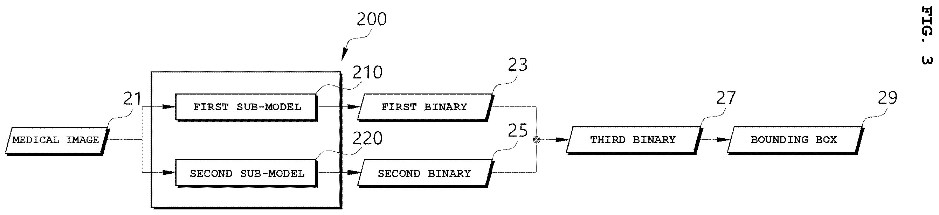

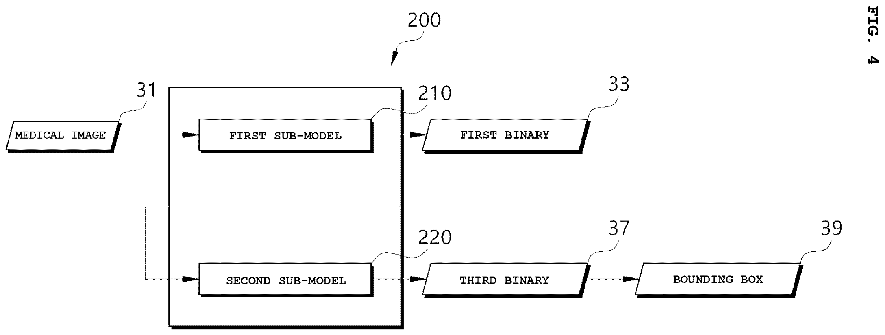

FIGS. 3 and 4 are block diagrams showing a process of searching for a blood collection candidate according to an alternative embodiment of the present disclosure.

Referring to FIG. 3, the processor 110 of the computing device 100 according to an alternative embodiment of the present disclosure may detect a blood vessel in a medical image 21 by inputting the medical image 21 to a deep learning model 200. In this case, the deep learning model 200 may include a first sub-model 210 for detecting at least one blood vessel present in the medical image 21 and a second sub-model 220 for detecting a superficial vein present in the medical image 21. More specifically, the processor 110 may detect at least one blood vessel present in the medical image 21 by binarizing the medical image 21 using the first sub-model 210. The processor 110 may generate a first binary 23 corresponding to the at least one blood vessel present in the medical image 21 by inputting the medical image 21 to the first sub-model 210. The processor 110 may detect a superficial vein present in the medical image 21 by binarizing the medical image 21 using the second sub-model 220. The processor 110 may generate a second binary 25 corresponding to the superficial vein present in the medical image 21 by inputting the medical image 21 to the second sub-model 220.

For example, when the computing device 100 acquires an infrared image of a blood vessel in the forearm as the medical image 21, the processor 110 may detect a blood vessel present in the infrared image by inputting the infrared image to the first sub-model 210 and binarizing the infrared image so that the blood vessel can be distinguished from the other regions in the infrared image. In other words, the first sub-model 210 may receive the infrared image and analyze its features, thereby generating the first binary 23 corresponding to the blood vessel by classifying a blood vessel region present in the infrared image as 1 and the other regions excluding the blood vessel present in the infrared image as 0. Furthermore, the processor 110 may detect a superficial vein present in the infrared image by inputting the infrared image to the second sub-model 220 and then binarizing it so that the superficial vein can be distinguished from the other regions in the infrared image. In other words, the second sub-model 220 may receive the infrared image and interpret its features, thereby generating the second binary 25 corresponding to the superficial vein by classifying a superficial vein region present in the infrared image as 1 and the other regions excluding the superficial vein present in the infrared image as 0.

Meanwhile, in this case, the first sub-model 210 may be pre-trained to detect all blood vessel regions in training images by using labels that mark all blood vessel regions, present in infrared images, with ground truth (GT). The training images may be medical images including infrared images of blood vessels. More specifically, in the training process, the first sub-model 210 may receive a training image, may detect a blood vessel region present in the image, and may perform binarization so that the detected region and the other regions can be distinguished from each other, thereby generating a binary corresponding to the blood vessel region. Furthermore, the first sub-model 210 may repeatedly perform a process of comparing the generated binary with labels and updating parameters based on the results of the comparison. Although the above-described example is a training process based on supervised learning, the first sub-model 210 may perform training through semi-supervised learning, unsupervised learning, self-supervised learning, or the like, other than supervised learning.

In addition, the second sub-model 220 may be pre-trained to detect a superficial vein by binarizing the medical image 21. The second sub-model 220 may be pre-trained to detect superficial veins in training images by using labels that mark superficial veins, such as the radial vein, the cubital vein, and the ulnar cubital vein, with ground truth (GT). The training images may be medical images including infrared images of blood vessels. More specifically, in the training process, the second sub-model 220 may receive a training image, may detect a superficial vein present in the image, and may perform binarization so that the superficial vein and the other regions can be distinguished from each other, thereby generating a binary corresponding to the superficial vein. Furthermore, the second sub-model 220 may repeatedly perform a process of comparing the generated binary with labels and updating parameters based on the results of the comparison. Although the above-described example is a training process based on supervised learning, the second sub-model 220 may perform training through semi-supervised learning, unsupervised learning, self-supervised learning, or the like, other than supervised learning.

The processor 110 may generate a third binary 27 to be used for determining a blood collection candidate by combining the first binary 23, which is the output of the first sub-model 210, and the second binary 25, which is the output of the second sub-model 220. More specifically, the processor 110 may apply a weight to the second binary 25 corresponding to a superficial vein present in the medical image 21. In this case, the application may be understood as a task of performing an operation such as multiplying the second binary 25 by a weight or adding a weight to the second binary 25. Furthermore, the processor 110 may generate the third binary 27 corresponding to a blood vessel present in the medical image 21 by combining the first binary 23 corresponding to at least one blood vessel present in the medical image 21 and the second binary to which the weight is applied. In this case, the combination may be understood as a task of performing an operation for combining the first binary 23 and the second binary to which the weight is applied.

For example, when the computing device 100 acquires an infrared image of a blood vessel in the forearm as the medical image 21, the processor 110 may multiply the pixel values of the second binary 25, representing a superficial vein region in the infrared image, by a weight. The processor 110 may generate the third binary 27 to be used for determining a blood collection candidate by multiplying the pixel values of the second binary, multiplied by the weight, by the pixel values of the first binary 23 representing at least one blood vessel region in the infrared image. The third binary 27 may be data that represent a superficial vein more prominently among all blood vessels while representing all the blood vessels present in the infrared image. As shown in FIG. 3(a), in the infrared image, the third binary 27 representing a blood vessel region may be represented in white, and the other regions excluding the third binary 27 may be represented in black.

The processor 110 may use an additional deep learning model to combine the first binary 23 and the second binary 25. More specifically, the processor 110 may generate the third binary 27 by inputting an image including the first binary 23 and an image including the second binary 25 to the additional model. In this case, the additional model may be pre-trained to detect blood collection candidate regions from training images by using labels that mark blood vessel regions, determined to be suitable for blood collection based on clinical criteria, with ground truth (GT). The training images may be medical images including infrared images of blood vessels, and may be binarized images so that a blood vessel region or superficial vein region and the other regions are distinguished from each other.

The processor 110 may more effectively derive a candidate suitable for blood collection when the third binary 27 generated through the above-described process is used to determine a blood collection candidate than when only the first binary 11 is used to determine a blood collection candidate. The superficial vein is a vein close to the skin, and corresponds to one of the blood vessels that are the main targets of blood collection. Accordingly, when the third binary 27 that simultaneously emphasizes a superficial vein while representing all blood vessels present in the medical image 21 is used, the processor 110 may select a blood collection candidate by analyzing the medical image 21 with a focus on blood vessels suitable for blood collection, such as a superficial vein, while excluding blood vessels that are not suitable for blood collection as much as possible.

Referring to FIG. 4, the processor 110 of the computing device 100 according to an alternative embodiment of the present disclosure may detect a superficial vein based on the blood vessel binary, extracted from the medical image 31, by using the second sub-model 220. The processor 110 may generate the third binary 39 to be used for determining a blood collection candidate by inputting the first binary 33, which is the output of the first sub-model 210, to the second sub-model 220. In this case, the first binary 33 is data generated by inputting the medical image 31 to the first sub-model 210, and may correspond to at least one blood vessel present in the medical image 31.

For example, when the computing device 100 acquires an infrared image of a blood vessel in the forearm as the medical image 31, the processor 110 may detect a blood vessel present in the infrared image by inputting the infrared image to the first sub-model 210 and binarizing it so that the blood vessel can be distinguished from the other regions in the infrared image. In other words, the first sub-model 210 may receive the infrared image and interpret its features, thereby generating the first binary 33 corresponding to the blood vessel by classifying a blood vessel region present in the infrared image as 1 and the other regions excluding the blood vessel present in the infrared image as 0. Furthermore, the processor 110 may detect a superficial vein present in the infrared image by inputting the infrared image, including the first binary 33, to the second sub-model 220 and binarizing it so that the superficial vein can be distinguished from the other regions in the infrared image. In other words, the second sub-model 220 may generate the third binary 39 corresponding to a superficial vein by inputting an infrared image in which a blood vessel region is binarized to 1 and the other regions are binarized to 0 and interpreting its features, thereby classifying a superficial vein region present in the infrared image as 1 and the other regions excluding the superficial vein present in the infrared image as 0. In this case, the third binary 39 generated as the output of the second sub-model 220 may be used to determine a blood collection candidate.

The second sub-model 220 may be pre-trained to detect superficial veins based on binarized medical images. The second sub-model 220 may be pre-trained to detect superficial veins in training images by using labels that mark superficial veins with ground truth (GT) in binarized images where blood vessel regions are binarized to 1 and the other regions are binarized to 0. The training images are medical images including infrared images of blood vessels, and may be images binarized such that a target region and the other regions are distinguished from each other. More specifically, in the training process, the second sub-model 220 may receive training images, may detect superficial veins, and may binarize the training images so that the superficial veins and other regions of the images can be distinguished from each other, thereby generating binaries corresponding to the superficial veins. Furthermore, the second sub-model 220 may repeatedly perform a process of comparing the generated binaries with the labels and updating parameters based on the results of the comparison. Although the above-described example is a training process based on supervised learning, the second sub-model 220 may perform training through semi-supervised learning, unsupervised learning, self-supervised learning, or the like, other than supervised learning.

When the third binary 37 generated via the above-described process is used to determine a blood collection candidate, the processor 110 may easily derive a superficial vein by using the deep learning model. The superficial vein is a vein close to the skin, and corresponds to one of the main targets of blood collection. Accordingly, when the third binary 37 in which a superficial vein is emphasized among all blood vessels present in the medical image 31 is used, the processor 110 may select a blood collection candidate by analyzing the medical image 31 with a focus on blood vessels suitable for blood collection, such as a superficial vein.

Meanwhile, the extraction of the bounding box 29 or 39 based on the third binary 27 or 37 in FIGS. 3 and 4 and the determination of the blood collection candidate based on the bounding box 29 or 39 equal to or larger than the predetermined size correspond to the extraction of the bounding box 19 and the determination of the blood collection candidate illustrated in FIG. 2 described above, so that descriptions thereof will be omitted below.

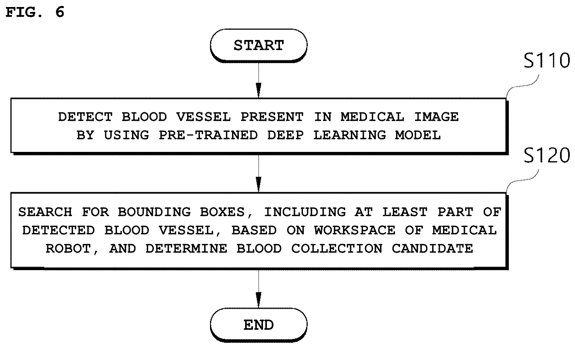

FIG. 6 is a flowchart showing a method for searching for a blood collection candidate according to one embodiment of the present disclosure.

Referring to FIG. 6, the computing device 100 according to one embodiment of the present disclosure may detect a blood vessel present in a medical image by using the pre-trained deep learning model in step S110. For example, the computing device 100 may generate a first binary corresponding to a blood vessel present in the medical image by using the first sub-model included in the deep learning model. The first sub-model may receive a medical image and perform binary segmentation for all blood vessels present in the medical image, thereby outputting a first binary representing a blood vessel present in the medical image. The computing device 100 may generate a second binary corresponding to a blood vessel present in the medical image by using the second sub-model included in the deep learning model. The first sub-model may receive a medical image and perform binary segmentation for a superficial vein present in the medical image, thereby outputting a second binary representing the superficial vein present in the medical image. The computing device 100 may apply a weight to the second binary, and may generate a third binary by combining the second binary, to which the weight is applied, and the first binary.

The computing device 100 may determine a blood collection candidate by searching for bounding boxes, including at least a part of the blood vessel detected via step S110, based on the workspace of the medical robot in step S120. The computing device 100 may search for bounding boxes present inside the blood vessel detected via step S110 and equal to or larger than a predetermined size within the operation limit angle of the medical robot. Furthermore, the computing device 100 may determine a bounding box equal to or larger than the predetermined size to be a blood collection candidate. For example, the computing device 100 may search for bounding boxes while rotating the third binary, representing the blood vessel detected in step S110, within the operation limit angle of the medical robot. The computing device 100 may select a bounding box equal to or larger than the predetermined size from among the bounding boxes found while rotating the detected blood vessel. The computing device 100 may repeatedly perform step S120 until the number of bounding boxes equal to or larger than the predetermined size becomes equal to or larger than a first reference value.

In addition, when it is predetermined that the largest size bounding box is to be selected from among the bounding boxes found while rotating the third binary representing the blood vessel detected in step S110, the computing device 100 may repeatedly perform step S120 until a bounding box, in which the number of pixels is smaller than or equal to a second reference value, is selected. Even when the largest bounding box is selected from among the bounding boxes present in the third binary, there is a probability that a bounding box having a size that is difficult to use for actual blood collection is to be selected, so that the computing device 100 can repeatedly perform step S120 until a bounding box, in which the number of pixels is smaller than or equal to the second reference value, is selected. In this case, the second reference value may be a value that is set by the manufacturer or user of the medical robot to achieve the purpose of blood collection. That is, the second reference value may be a value that is determined in advance by the manufacturer or user of the medical robot based on the clinical experience in which a thick and long blood vessel is suitable for blood collection. The second reference value may be modified or changed by an external input that is applied through communication with the medical robot itself, a client terminal, or the like.

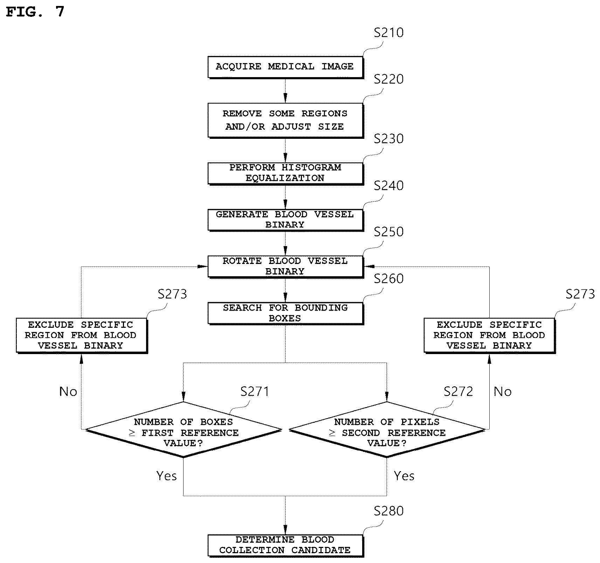

FIG. 7 is a flowchart showing a process of searching for a blood collection candidate according to one embodiment of the present disclosure.

Referring to FIG. 7, the computing device 100 according to one embodiment of the present disclosure may acquire a medical image in step S210. For example, the computing device 100 may receive a medical image of a blood vessel through cloud communication with a picture archiving and communication system. The computing device 100 may be included as a component of a medical robot, and may generate a medical image of a blood vessel via a camera. In this case, the camera may include an infrared camera, an ultrasonic camera, an RGB camera, etc.

In step S220, the computing device 100 may remove some regions from the medical image acquired via step S210, or may adjust the size of the medical image acquired via step S210. For example, when an infrared image having a size of 1920 × 1200 is acquired via step S210, the computing device 100 may adjust the size of the image to 1600 × 1200 by removing a region where noise unnecessary for blood vessel detection is present from the infrared image. Then, the computing device 100 may reduce the image, which has been adjusted to 1600 × 1200 by removing the noise region, to 400 × 300 to meet the current ratio according to the input specifications of the deep learning model.

In step S230, the computing device 100 may perform histogram equalization on the medical image processed via step S220. More specifically, the computing device 100 may divide the medical image into a plurality of tiles and perform histogram equalization for each of the tiles. Furthermore, the computing device 100 may adjust the contrast of the medical image so that a blood vessel present in the medical image is emphasized. Through the equalization, the medical image may be processed such that a blood vessel present in the medical image is visually represented more prominently than the other regions.

The computing device 100 may generate a blood vessel binary by inputting the medical image, processed via steps S220 to S230, to the deep learning model in step S240. The computing device 100 may detect a blood vessel by inputting the medical image to the deep learning model and binarizing the medical image so that a blood vessel region and the other regions can be distinguished from each other. Furthermore, the computing device 100 may perform an operation for selecting a blood collection candidate based on the blood vessel binary.

The computing device 100 may rotate the blood vessel binary, generated via step S240, within the operation limit angle of the medical robot in step S250. Furthermore, the computing device 100 may search for bounding boxes adjacent to the blood vessel binary while rotating the blood vessel binary in step S260. In this case, each of the bounding boxes is a part of the blood vessel binary, and may be understood as a blood vessel region that can be a blood collection candidate. Furthermore, the computing device 100 may select a bounding box equal to or larger than a predetermined size from among the found bounding boxes as a blood collection candidate. For example, in the case where the insertion angle of a needle is limited due to the invasive structure of the medical robot, when a blood collection candidate is determined without considering the insertion angle, the medical robot may not perform a blood collection operation. Accordingly, the computing device 100 may extract blood vessel regions available for candidates for blood collection by searching for bounding boxes within the blood vessel binary while rotating the blood vessel binary within the insertion angle of the needle according to the structure of the medical robot. The computing device 100 may select a bounding box equal to or larger than a size suitable for blood collection from among the found bounding boxes. In this case, the size suitable for blood collection may be a value that is set in advance based on the clinical experience of the manufacturer or user of the medical robot.

The computing device 100 may determine whether to re-perform an operation process for selecting a bounding box based on the number of selected boxes or the number of pixels from among the bounding boxes found via step S250. More specifically, the computing device 100 may determine whether the number of bounding boxes equal to or larger than the predetermined size is equal to or larger than a first reference value (e.g., five) in step S271. When the number of bounding boxes is equal to or larger than the first reference value, the computing device 100 may determine the boxes, selected as bounding boxes equal to or larger than the predetermined size, to be blood collection candidates in step S280. When the number of bounding boxes is smaller than the first reference value, the computing device 100 may re-perform steps S250 and S260 for selecting a bounding box. Meanwhile, when it is predetermined that the largest bounding box is to be selected from among the bounding boxes found while rotating the blood vessel binary, the computing device 100 may determine whether the number of pixels of the box selected as the largest bounding box is smaller than or equal to a second reference value (e.g., 20 pixels) in step S272. When the number of pixels is smaller than or equal to the second reference value, the computing device 100 may determine the selected bounding box to be a blood collection candidate until the box in which the number of pixels is smaller than or equal to the second reference value is selected in step S280. When the number of pixels exceeds the second reference value, the computing device 100 may re-perform steps S250 and S260 for selecting a bounding box. When it is predetermined that the largest bounding box is to be selected from among the bounding boxes found while rotating the blood vessel binary, above-described steps S271 and S272 may be performed in parallel or selectively.

When it is determined that steps S250 and S260 for selecting a bounding box are to be re-performed, the computing device 100 may perform re-searching and selection within the blood vessel binary excluding a specific region in step S273. When a previous search area is included in the re-searching process, there is a possibility that a redundant search area is generated, and thus, an unnecessary operation is performed. Accordingly, the computing device 100 may re-perform steps S250 and S260 while excluding predetermined numbers of adjacent pixels in the forward, backward, left, and right directions in the area of a previous search blood vessel binary from searching. For example, the computing device 100 may exclude up to ten pixels in front of and behind a previous search area from a re-search area for bounding box re-searching and selection. The above-described numerical values are only examples, and thus, the present disclosure is not limited thereto.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

## Claims

1. A method for searching for a blood collection candidate, the method being performed by a computing device including at least one processor, the method comprising:
detecting a blood vessel present in a medical image by using a pre-trained deep learning model; and
searching for bounding boxes, including at least a part of the detected blood vessel, based on a workspace of a medical robot, and determining a blood collection candidate.

2. The method of claim 1, wherein detecting the blood vessel present in the medical image by using the pre-trained deep learning model comprises:
generating a first binary corresponding to a blood vessel present in the medical image by using a first sub-model included in the deep learning model; and
generating a second binary corresponding to a blood vessel present in the medical image by using a second sub-model included in the deep learning model;
wherein the second sub-model is pre-trained to detect a superficial vein by binarizing a medical image.

3. The method of claim 2, wherein detecting the blood vessel present in the medical image by using the pre-trained deep learning model further comprises:
applying a weight to the second binary; and
generating a third binary to be used for determining the blood collection candidate by combining the second binary to which the weight is applied and the first binary.

4. The method of claim 1, wherein searching for the bounding boxes, including at least a part of the detected blood vessel, based on the workspace of the medical robot, and determining the blood collection candidate comprises:
searching for bounding boxes present inside the detected blood vessel and equal to or larger than a predetermined size within an operation limit angle of the medical robot; and
determining the bounding box equal to or larger than the predetermined size to be a blood collection candidate.

5. The method of claim 4, wherein searching for the bounding boxes including at least a part of the detected blood vessel and equal to or larger than the predetermined size within the operation limit angle of the medical robot comprises:
searching for bounding boxes while rotating the detected blood vessel within the operation limit angle of the medical robot; and
selecting a bounding box equal to or larger than a predetermined size from among the bounding boxes found while rotating the detected blood vessel.

6. The method of claim 5, wherein searching for the bounding boxes while rotating the detected blood vessel within the operation limit angle of the medical robot and selecting the bounding box equal to or larger than the predetermined size from among the bounding boxes found while rotating the detected blood vessel are repeatedly performed until a number of selected bounding boxes becomes equal to or larger than a first reference value.

7. The method of claim 1, wherein detecting the blood vessel present in the medical image by using the pre-trained deep learning model comprises:
generating a first binary corresponding to a blood vessel present in the medical image by using a first sub-model included in the deep learning model; and
generating a third binary to be used for determining the blood collection candidate based on the first binary by using a second sub-model included in the deep learning model;
wherein the second sub-model is pre-learned to detect a superficial vein based on a binarized medical image.

8. The method of claim 1, wherein,
when it is predetermined that a largest bounding box is selected from among the bounding boxes found while rotating the detected blood vessel,
searching for the bounding boxes while rotating the detected blood vessel within the operation limit angle of the medical robot and selecting the bounding box equal to or larger than the predetermined size from among the bounding boxes found while rotating the detected blood vessel are repeatedly performed until a bounding box, in which a number of pixels is smaller than or equal to a second reference value, is selected.

9. The method of claim 1, further comprising:
dividing the medical image into a plurality of tiles and performing histogram equalization for each of the tiles.

10. The method of claim 1, further comprising:
adjusting contrast of the medical image so that a blood vessel present in the medical image is emphasized.

11. A computer program stored in a computer-readable storage medium, the computer program performing operations for searching for a blood collection candidate when executed on one or more processors, wherein the operations comprise operations of:
detecting a blood vessel present in a medical image by using a pre-trained deep learning model; and
searching for bounding boxes, including at least a part of the detected blood vessel, based on a workspace of a medical robot, and determining a blood collection candidate.

12. A computing device for searching for a blood collection candidate, the computing device comprising:
a processor including at least one core;
memory including program codes executable on the processor; and
a network unit for acquiring a medical image;
wherein the processor:
detects a blood vessel present in a medical image by using a pre-trained deep learning model; and
searches for bounding boxes, including at least a part of the detected blood vessel, based on a workspace of a medical robot, and determines a blood collection candidate.
